# EUROPÄISCHE PATENTSCHRIFT

(11) **EP 1 424 044 B1**
(45) Veröffentlichungstag und Bekanntmachung des Hinweises auf die Patenterteilung: **23.07.2008**
(21) Anmeldenummer: 03025229.0
(22) Anmeldetag: 05.11.2003
(51) Int. Cl.: A61F 2/00, A61F 2/06

(54) **Künstlicher Endosphinkter**
Prosthetic endosphincter
Prothèse d'endosphincter

(30) Priorität: 30.11.2002 DE 10256027
(43) Veröffentlichungstag der Anmeldung: 02.06.2004
(73) Patentinhaber: Chaussy, Christian, Prof. Dr. med., 82064 Strasslach (DE); Thüroff, Stefan, Dr. med., 81545 München (DE); Schmitt, Klaus, 73630 Remshalden-Grunbach (DE)
(72) Erfinder: Chaussy, Christian, Prof. Dr. med., 82064 Strasslach (DE); Thüroff, Stefan, Dr. med., 81545 München (DE); Schmitt, Klaus, 73630 Remshalden-Grunbach (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- WO-A-98/32412
- WO-A-99/13801
- DE-U- 29 507 519
- US-A- 4 968 294
- US-A- 5 800 339

## Beschreibung

### Stand der Technik

Die Erfindung betrifft einen künstlichen Endosphinkter für die Urethra, mit einem Rückhalteteil und einem von außen durch Druckausübung manuell betätigbaren Ventil.

Ein derartiger künstlicher Endosphinkter ist beispielsweise aus der DE-U-295 07 519 bekannt.

Zur Inkontinenztherapie beim Mann nach einer Prostataresektion und beim "alten inkontinenten Mann" finden künstliche Endosphinkter Anwendung. Die Harnröhre liegt zwischen den beiden Schwellkörpern an der Unterseite des Penis. Mit Daumen und Zeigefinger drückt man von unten her die Harnröhre gegen die darüber liegenden zueinander parallel liegenden Schwellkörper. Durch den Druck auf die Harnröhre wird auch ein Druck auf das manuell betätigbare Ventil ausgeübt, wodurch dieses sich öffnen soll. Da die Harnröhre etwa zu 270° von den Schwellkörpern umgeben ist, muss der künstliche Endosphinkter so positioniert werden, dass das Ventil durch einen von Außen manuell aufgebauten Druck auf einen relativ geringen Winkelbereich der Harnröhre von etwa 90° geöffnet werden kann.

Aus der DE-U-295 07 519 ist ein künstlicher Endosphinkter bekannt, dessen Rückhalteteil zwei verschiedene Durchmesser aufweist, wobei ein Ventilkörper an demjenigen Rückhalteteilabschnitt befestigt ist, der den kleineren Durchmesser aufweist. Der künstliche Endosphinkter weist einen Haltebereich zwischen dem Rückhalteteil mit kleinerem Durchmesser und dem Rückhalteteil mit größerem Durchmesser auf. Das bekannte Rückhalteteil wird in einer Einführ-Konfiguration in ein Hohlorgan eingeführt und dann durch die Körpertemperatur expandiert. Weiterhin weist der Ventilkörper zwei Dichtlippen auf, die zwischen sich eine Schlitzöffnung einschließen. Die Dichtlippen müssen in der Urethra parallel zur Betätigungsrichtung angeordnet sein und daher beim Platzieren des künstlichen Endosphinkters richtig orientiert werden. Gelingt dies nicht, kann es sein, dass die Dichtlippen quer oder senkrecht zur Betätigungsrichtung zu liegen kommen. In diesem Fall kann das Ventil mittels Fingerdruck nicht mehr geöffnet werden, sondern wird vielmehr noch fester verschlossen.

### Aufgabe der Erfindung

Aufgabe der vorliegenden Erfindung ist es deshalb, einen künstlichen Endosphinkter der eingangs genannten Art dahingehend weiterzubilden, dass er temperaturunabhängig expandierbar ist und/oder ein Ventil aufweist, das lageunabhängig geöffnet werden kann.

### Gegenstand der Erfindung

Erfindungsgemäß wird diese Aufgabe dadurch gelöst, dass das Rückhalteteil als selbstexpandierender Stent ausgebildet ist.

Wenn der Stent selbstexpandierend ist, kann auf eine Expansion mittels eines Ballons oder unter Einsatz von erhöhter Temperatur verzichtet werden. Das Einbringen des Stents wird dadurch erheblich vereinfacht. Zum Einführen kann der Stent zusammen gedrückt werden, indem er beispielsweise gelängt wird. Nach dem Einführen dehnt er sich von selbst wieder aus, im Wesentlichen auf die Form vor dem Zusammendrücken. Der selbstexpandierbare Stent ist flexibel, so dass er der Krümmung der Urethra folgen kann.

Bei einer bevorzugten Ausführungsform ist der Stent ein aus mindestens einem Filament hergestelltes tubuläres Geflecht. Mit einem solchen tubulären Geflecht kann ein selbstexpandierender Stent besonders einfach realisiert werden.

Vorzugsweise kann der erfindungsgemäße künstliche Endosphinkter gekürzt werden, zum Beispiel durch einfaches Abschneiden des Stents. Dafür sind am Stent Abschnitte vorgesehen, in denen sich kein tubuläres Geflecht befindet. An diesen geflechtfreien Stellen kann der Stent einfach abgeschnitten werden. Durch diese Möglichkeit kann der künstliche Endosphinkter zum einen individuell auf jeden Patienten eingestellt und angepasst werden und zum anderen kann ein relativ kurzes Rückhalteteil zur Inkontinenztherapie Anwendung finden. Das ungekürzte Rückhalteteil kann bei akuter Harnverhaltung bzw. Harnsperre auch zur Ausschaltung des funktionierenden Sphinkters angewendet werden.

In einer bevorzugten Ausgestaltung der Erfindung weist das mindestens eine Filament einen runden Querschnitt auf. Diese Maßnahme hat den Vorteil, dass sich das Gewebe der Urethra gut an den Stent anschmiegen kann und keine Verletzung des Gewebes beim Einbringen und Entfernen des Stents entsteht. Je nach Anwendung des Stents kann es aber auch vorteilhaft sein, ein Filament mit anderem Querschnitt, z.B. rechteckig, dreieckig oder ellipsenförmig, zu wählen.

In einer besonders bevorzugten Ausgestaltung der Erfindung ist das mindestens eine Filament aus Kunststoff oder Metall gefertigt. Ein mit diesen Materialien gefertigter Stent besitzt eine Art "Formgedächtnis" und zeigt daher einen sogenannten "Memory-Effekt". Das tubuläre Geflecht nimmt nach einer Komprimierung immer wieder seinen expandierten Ausgangszustand an. Das tubuläre Geflecht kann also zur Einbringung des Stents so komprimiert werden, dass der Stent ein kleines Lumen aufweist. Nach der Einbringung in die Urethra expandiert der Stent selbständig, so dass ein großes Lumen des Stents entsteht und Urin ungestört zum Ventil gelangt.

Es ist vorteilhaft, wenn der künstliche Endosphinkter röntgendichte Markierungen, insbesondere an seinen Enden, aufweist. Die Platzierung des künstlichen Endosphinkters kann bei dieser Maßnahme durch Röntgenaufnahmen kontrolliert werden. Es kann auch vorgesehen sein, die röntgendichten Markierungen ausschließlich am Stent anzubringen.

Wenn der Stent zylindrisch ausgebildet ist, kann der Endosphinkter besonders leicht wieder entfernt werden, da der Stent keine Aufbauchung aufweist, die ein Verschieben in der Urethra verhindern könnte. Auch kann ein zylindrischer Stent besonders klein komprimiert werden und somit wenig Irritationen verursachend eingeführt werden.

Wenn der Stent mindestens zwei zylindrische Abschnitte mit unterschiedlichem Radius aufweist, kann die Rückstellkraft des Stents bereichsweise eingestellt werden, sodass z. B. im Bereich der Prostata oder des an die Blase anschließenden Ringmuskels ein hinreichend großes Lumen des Endosphinkters sicher gestellt wird. Der Stent kann je nach Anwendung andere vorteilhafte Formen, beispielsweise bauchige Formen, und abschnittsweise Lumenerweiterungen aufweisen.

In einer besonders bevorzugten Ausführungsform ist das tubuläre Geflecht sowohl innen als auch außen von einer Beschichtung umgeben, die auf der Innenoberfläche glatt ist und auf der Außenoberfläche die Geflechtstruktur nachbildet. Die glatte Innenoberfläche ermöglicht ein gutes Fließen von Urin im Stent und verhindert Ablagerungen (Inkrustationen). Die Beschichtung auf der Außenoberfläche des Stents ist vorteilhafterweise dünner als der Durchmesser des Filaments, so dass die Struktur des tubulären Geflechts erhalten bleibt und der Stent dadurch ortsfest in der Urethra gehalten werden kann. Insbesondere kann vorgesehen sein, dass das tubuläre Geflecht einen Schlauch umgibt, auf dessen Oberfläche eine das tubuläre Geflecht auf dem Schlauch haltende Beschichtung aufgetragen ist. Die das tubuläre Geflecht auf dem Schlauch haltende Beschichtung ist vorzugsweise aus demselben Material wie der Schlauch. Dadurch kann eine besonders gute, insbesondere materialschlüssige, Verbindung der beiden Beschichtungen erzielt werden.

Bei einer bevorzugten Weiterbildung mündet der Schlauch in eine Ventilaufnahme. Durch diese Maßnahme wird die Ventilaufnahme am Rückhalteteil gehalten. Außerdem ist es möglich, den Schlauch und die Ventilaufnahme einstückig aus demselben Material auszubilden.

Wenn der Übergangsbereich zwischen Ventilaufnahme und Schlauch konisch ausgebildet ist, kann die Ventilaufnahme mit geringerem Durchmesser ausgebildet werden als der Schlauch. Dieser Übergangsbereich ermöglicht es, dass der Schlauch und damit der Stent in etwa auf den Durchmesser der Ventilaufnahme komprimiert werden können. Durch diese Maßnahme wird der künstliche Endosphinkter bei der Platzierung nicht beschädigt.

Bei einer Weiterbildung ist der Übergangsbereich von dem tubulären Geflecht ummantelt. Durch diese Maßnahme lässt sich der künstliche Endosphinkter leichter wieder aus der Urethra entfernen.

Wenn sich die Filamente des tubulären Geflechts entlang des Übergangsbereichs zur Ventilaufnahme hin verjüngen, wird die Elastizität und Flexibilität des Übergangsbereichs auch am ventilseitigen Ende aufrecht erhalten. Ein unbeweglicher Materialring aufgrund von zusammengepressten gleichmäßig dicken Filamenten wird vermieden.

Besonders vorteilhaft ist es, wenn der Schlauch und die Ventilaufnahme einstückig, insbesondere einstückig aus Silikon, ausgebildet sind. Die einstückige Ausbildung ermöglicht ein kostengünstiges Herstellen des Endosphinkters. Der Endosphinkter muss nicht aufwändig zusammengebaut werden. Silikon ist ein hochflexibles Material, das bei der Platzierung verformt werden kann und nach der Platzierung in seine Ursprungsform selbsttätig zurückkehrt. Insbesondere bei der Ventilaufnahme ist es wichtig, dass sie nach Betätigen des Ventils wieder in ihre Ursprungsposition zurückkehrt, damit das Ventil nach einer gewollten Öffnung selbsttätig schließen kann.

Bei einer besonders bevorzugten Ausführungsform ist das Ventil als selbst schließendes Kreuzschlitzventil ausgebildet. Die Verwendung eines Kreuzschlitzventils hat den großen Vorteil, dass das Ventil in nahezu jeder Lage in der Urethra betätigt werden kann. Bei der Platzierung des künstlichen Endosphinkters muss nicht auf die Orientierung in der Urethra geachtet werden. Ein unbeabsichtigtes Verschließen des Ventils bei Druckausübung auf die Urethra ist ausgeschlossen. Fehlfunktionen sind nahezu unmöglich.

Bei einer besonders bevorzugten Ausführungsform weist das Kreuzschlitzventil einen hohlzylindrischen Befestigungsabschnitt und einen dem Rückhalteteil zugewandten Schließabschnitt auf. Mit dem hohlzylindrischen Befestigungsabschnitt wird das Ventil in der Ventilaufnahme gehalten. Dabei liegt der Befestigungsabschnitt mit seiner Außenoberfläche an der Innenoberfläche der Ventilaufnahme an. Das Ventil ist mit dem Befestigungsabschnitt formschlüssig in der Ventilaufnahme gehalten. Wenn der Schließabschnitt des Kreuzschlitzventils dem Rückhalteteil zugewandt ist, erfolgt ein besseres Schließen des Kreuzschlitzventils bei Flüssigkeitsdruck in axialer Richtung des künstlichen Endosphinkters. Je mehr Druck auf den Schließabschnitt ausgeübt wird, desto besser schließt das Ventil.

Bei einer bevorzugten Weiterbildung weist das Kreuzschlitzventil Druckaufnahmeflächen auf. Diese Druckaufnahmeflächen, die statischen Druck von vier Seiten aufnehmen können, stellen ein gutes Schließen der Dichtungslippen sicher.

Wenn die Druckaufnahmeflächen in Dichtungslippen münden, werden die Druckaufnahmeflächen besonders gut ausgenutzt und erfolgt ein zuverlässiges Schließen des Ventils.

Vorzugsweise ist das Kreuzschlitzventil aus Silikon hergestellt. Wenn das Kreuzschlitzventil und die Ventilaufnahme aus demselben Material hergestellt sind, werden sie bei Druck auf die Urethra gleichmäßig verformt. Aufgrund der elastischen Eigenschaften von Silikon kehren das Ventil und die Ventilaufnahme in ihre Ursprungsposition zurück, wenn kein Druck ausgeübt wird. Dadurch erfolgt ein automatisches Schließen des Kreuzschlitzventils, wenn kein radialer Druck auf die Urethra ausgeübt wird.

Weitere Merkmale und Vorteile der Erfindung ergeben sich aus der nachfolgenden Beschreibung eines Ausführungsbeispiels der Erfindung, anhand der Figuren der Zeichnung, die erfindungswesentliche Einzelheiten zeigen, und aus den Ansprüchen. Die einzelnen Merkmale können je einzeln für sich oder zu mehreren in beliebiger Kombination bei einer Variante der Erfindung verwirklicht sein.

### Zeichnung

Ein Ausführungsbeispiel des erfindungsgemäßen künstlichen Endosphinkters ist in der schematischen Zeichnung dargestellt und wird in der nachfolgenden Beschreibung erläutert. Es zeigt:
- **Fig. 1**: einen Teil-Längsschnitt durch einen künstlichen Endosphinkter;
- **Fig. 2**: eine perspektivische Ansicht eines geschlossenen Ventils; und
- **Fig. 3**: eine Vorderansicht auf ein geöffnetes Ventil.

### Beschreibung des Ausführungsbeispiels

Die Figuren sind sehr schematisch dargestellt, um die wesentlichen erfinderischen Merkmale zu verdeutlichen. In den Darstellungen sind die Dimensionen nur beispielhaft und nicht maßstäblich zu verstehen.

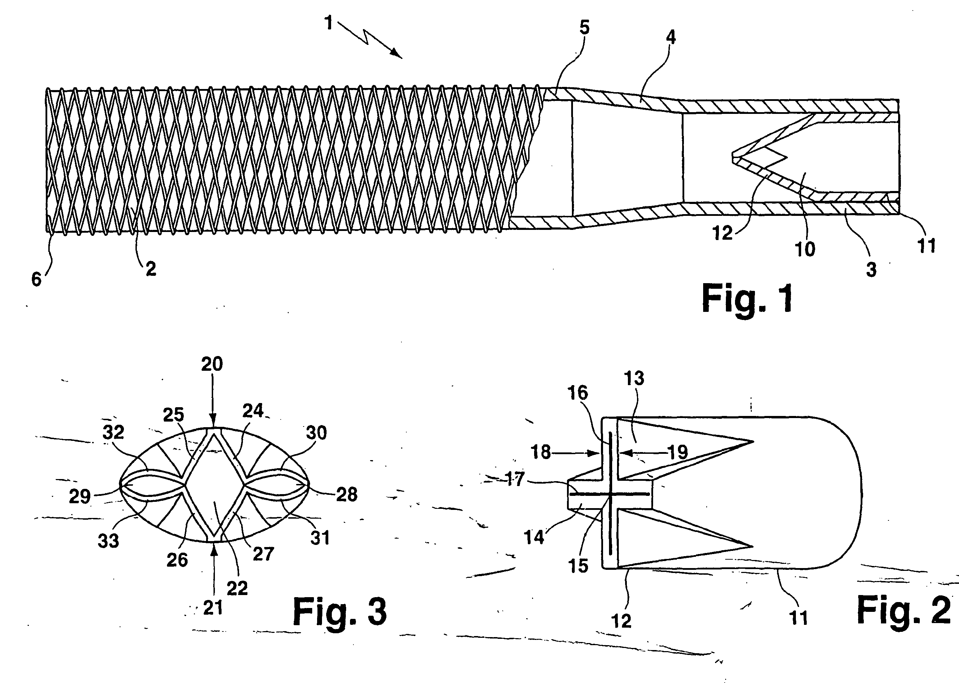

**Fig. 1** zeigt einen Teil-Längsschnitt durch einen künstlichen Endosphinkter **1**. Der künstliche Endosphinkter 1 weist ein als Stent ausgebildetes Rückhalteteil **2**, eine Ventilaufnahme **3** und einen die Ventilaufnahme 3 mit dem Rückhalteteil 2 verbindenden Übergangsbereich **4** auf. Der Innendurchmesser der Ventilaufnahme 3 ist geringer als der Innendurchmesser des Rückhalteteils 2. Der Übergangsbereich 4 ist konisch ausgebildet, um diesen Unterschied der Innendurchmesser auszugleichen. Der künstliche Endosphinkter 1 ist schlauchförmig ausgebildet, wobei der Schlauch **5** im Bereich des Rückhalteteils 2 durch ein tubuläres Geflecht **6** ummantelt ist. Das tubuläre Geflecht 6 ist durch eine dünne, nicht gezeigte, Beschichtung umgeben, die das tubuläre Geflecht 6 auf dem Schlauch 5 hält. Die Schichtdicke der Beschichtung ist so dünn gewählt, dass die Struktur des Geflechts 5 erhalten bleibt. Dies bewirkt eine rutschsichere Halterung in der Urethra. Das Rückhalteteil 2 kann gelängt werden, wodurch sich der Innendurchmesser des Rückhalteteils 2 dem Innendurchmesser der Ventilaufnahme 3 annähern lässt. Die Innenoberfläche des Schlauchs 5 ist glatt ausgebildet, um Ablagerungen zu verhindern. Der Übergangsbereich 4 und die Ventilaufnahme 3 weisen kein tubuläres Geflecht auf. In der Ventilaufnahme 3 ist ein Kreuzschlitzventil **10** gehalten. Das Kreuzschlitzventil 10 umfasst einen hohlzylindrischen Befestigungsabschnitt **11** und einen Schließabschnitt **12**. Der Schließabschnitt 12 ist dem Rückhalteteil zugewandt. In der gezeigten Stellung ist das Kreuzschlitzventil 10 geschlossen. Wird auf die Ventilaufnahme 3 radial Druck ausgeübt, öffnet sich der Schließabschnitt 12 und gibt eine Öffnung frei, durch die Urin ausströmen kann.

**Fig. 2** zeigt eine perspektivische Ansicht des Kreuzschlitzventils 10. An den zylindrischen Befestigungsabschnitt 11 schließt sich der Schließabschnitt 12 an. Im Schließabschnitt 12 sind Druckaufnahmeflächen **13** vorgesehen, die in Dichtlippen **14** münden. Zwischen den Dichtlippen 14 befindet sich der Kreuzschlitz **15**, der in der Fig. 2 geschlossen gezeichnet ist. Bei radialem Druck auf das Kreuzschlitzventil 10 öffnet sich der Kreuzschlitz 15 wenigstens entlang einer der Linien **16**, **17**. Bei dem erfindungsgemäßen Kreuzschlitzventil 10 ist es nahezu gleichgültig, an welcher Stelle radialer Druck auf das Kreuzschlitzventil 10 ausgeübt wird, um den Kreuzschlitz 15 zu öffnen. Aus der Fig. 2 ist ersichtlich, dass, wenn Flüssigkeit auf den Schließabschnitt 12 trifft, Druck auf die Druckaufnahmeflächen 13, beispielsweise in Richtung der Pfeile **18, 19** ausgeübt wird. Dadurch werden die Dichtlippen 14 stärker aneinander gepresst, sodass das Kreuzschlitzventil 10 sicher verschlossen wird.

**Fig. 3** zeigt eine Vorderansicht auf ein geöffnetes Ventil 10. Bei radialem Druck in Richtung der Pfeile **20, 21** wird eine Hauptöffnung **22** zwischen den Dichtlippen **24 bis 27** und zwei Nebenöffnungen **28, 29** zwischen den Dichtlippen **30, 31** bzw. **32**, **33** freigegeben. Wird kein radialer Druck mehr auf das Kreuzschlitzventil 10 ausgeübt, kehrt es in seine Ausgangsstellung zurück und die Dichtlippen verschließen die Öffnungen 22, 28, 29.

Bei einem Künstlichen Endosphinkter 1 für die Urethra, mit einem Rückhalteteil 2 und einem von außen durch Druckausübung manuell betätigbaren Ventil 10 ist das Rückhalteteil 2 als selbstexpandierender Stent ausgebildet. Dies ermöglicht neben einer schonenden, einfachen und sicheren Platzierung eine zuverlässige und einfache Handhabung des künstlichen Endosphinkters 1.

## Patentansprüche

1. Künstlicher Endosphinkter (1) für die Urethra, mit einem Rückhalteteil (2) und einem von außen durch Druckausübung manuell betätigbaren Ventil (10), **dadurch gekennzeichnet, dass** das Ventil als selbstschließendes Kreuzschlitzventil (10) ausgebildet ist.

2. Endosphinkter nach Anspruch 1, **dadurch gekennzeichnet, dass** das Kreuzschlitzventil (10) einen hohlzylindrischen Befestigungsabschnitt (11) und einen dem Rückhalteteil (2) zugewandten Schließabschnitt (12) aufweist.

3. Endosphinkter nach Anspruch 1 oder 2, **dadurch gekennzeichnet, dass** das Kreuzschlitzventil (10) Druckaufnahmeflächen (13) aufweist.

4. Endosphinkter nach Anspruch 3, **dadurch gekennzeichnet, dass** die Druckaufnahmeflächen (13) in Dichtungslippen (14; 24-27) münden.

5. Endosphinkter nach einem der Ansprüche 1 bis 4, **dadurch gekennzeichnet, dass** das Kreuzschlitzventil (10) aus Silikon hergestellt ist.

6. Endosphinkter nach einem der vorangehenden Ansprüche, **dadurch gekennzeichnet, dass** der Stent ein aus mindestens einem Filament hergestelltes tubuläres Geflecht (6) ist.

7. Endosphinkter nach Anspruch 6, **dadurch gekennzeichnet, dass** das Rückhalteteil mindestens einen Abschnitt ohne tubuläres Geflecht aufweist.

8. Endosphinkter nach Anspruch 6 oder 7, **dadurch gekennzeichnet, dass** das mindestens eine Filament einen runden Querschnitt aufweist.

9. Endosphinkter nach einem der Ansprüche 6 bis 8, **dadurch gekennzeichnet, dass** das mindestens eine Filament aus Kunststoff oder Metall gefertigt ist.

10. Endosphinkter nach einem der Ansprüche 6 bis 9, **dadurch gekennzeichnet, dass** das tubuläre Geflecht (6) sowohl innen als auch außen von einer Beschichtung umgeben ist, die auf der Innenoberfläche glatt ist und auf der Außenoberfläche die Geflechtsstruktur nachbildet.

11. Endosphinkter nach Anspruch 10, **dadurch gekennzeichnet, dass** die Innenbeschichtung als Schlauch (5) ausgebildet ist, der von dem tubulären Geflecht (6) umgeben ist.

12. Endosphinkter nach Anspruch 11, **dadurch gekennzeichnet, dass** der Schlauch (5) in eine Aufnahme (3) des Kreuzschlitzventils mündet.

13. Endosphinkter nach Anspruch 12, **dadurch gekennzeichnet, dass** ein Übergangsbereich (4) zwischen der Ventilaufnahme (3) und dem Schlauch (5) konisch ausgebildet ist.

14. Endosphinkter nach Anspruch 13, **dadurch gekennzeichnet, dass** der Übergangsbereich (4) von dem tubulären Geflecht ummantelt ist.

15. Endosphinkter nach Anspruch 14, **dadurch gekennzeichnet, dass** sich die Filamente des tubulären Geflechts zur Ventilaufnahme hin verjüngen.

16. Endosphinkter nach einem der Ansprüche 12 bis 15, **dadurch gekennzeichnet, dass** der Schlauch (5) und die Ventilaufnahme (3) einstückig, insbesondere einstückig aus Silikon, ausgebildet sind.

17. Endosphinkter nach einem der vorhergehenden Ansprüche, **dadurch gekennzeichnet, dass** der Stent zylindrisch ausgebildet ist.

18. Endosphinkter nach einem der vorhergehenden Ansprüche, **dadurch gekennzeichnet, dass** der Stent mindestens zwei zylindrische Abschnitte mit unterschiedlichem Radius aufweist.

19. Endosphinkter nach einem der vorhergehenden Ansprüche, **dadurch gekennzeichnet, dass** der künstliche Endosphinkter röntgendichte Markierungen aufweist.

20. Endosphinkter nach einem der vorhergehenden Ansprüche, **dadurch gekennzeichnet, dass** das Rückhalteteil (2) als temperaturunabhängig selbstexpandierender Stent ausgebildet ist.

## Claims

1. An artificial endosphincter (1) for the urethra, comprising a retaining piece (2) and a valve (10), manually operable from the outside through application of pressure, wherein the valve is provided as a self closing cross slot valve (10).

2. An endosphincter according to claim 1, wherein the cross slot valve (10) comprises a hollow cylindrical mounting section (11) and a closing section (12), facing towards the retaining piece (2).

3. An endosphincter according to claim 1 or 2, wherein the cross slot valve (10) comprises pressure receiving surfaces (13).

4. An endosphincter according to claim 3, wherein the pressure receiving surfaces (13) transition into seal lips (14; 24-27).

5. An endosphincter according to one of the claims 1 through 4, wherein the cross slot valve (10) is made of silicon.

6. An endosphincter according to one of the preceding claims, wherein the stent is a tubular meshwork (6), made of at least one filament.

7. An endosphincter according to claim 6, wherein the retaining piece comprises at least one section without tubular meshwork.

8. An endosphincter according to claims 6 or 7, wherein the at least one filament comprises a round cross section.

9. An endosphincter according to one of the claims 6 through 8, wherein the at least one filament is made of plastic or metal.

10. An endosphincter according to one of the claims 6 through 9, wherein the tubular meshwork (6) is surrounded by a surface coating on the inside and on the outside, which is smooth on the inner surface and replicates the meshwork structure on the outer surface.

11. An endosphincter according to claim 10, wherein the inner coating is provided as a hose (5), surrounded by the tubular meshwork (6).

12. An endosphincter according to claim 11, wherein the hose (5) leads into a receiver (3) of the cross slot valve.

13. An endosphincter according to claim 12, wherein a transition section (4) between the valve receiver (3) and the hose (5) is provided conical.

14. An endosphincter according to claim 13, wherein the transition section (4) is surrounded by the tubular meshwork.

15. An endosphincter according to claim 14, wherein the filaments of the tubular meshwork taper towards the valve receiver.

16. An endosphincter according to one of the claims 12 through 15, wherein the hose (5) and the valve receiver (3) are provided integral, in particular provided integral from silicon.

17. An endosphincter according to one of the preceding claims, wherein the stent is provided cylindrical.

18. An endosphincter according to one of the preceding claims, wherein the stent comprises at least two cylindrical sections with different radii.

19. An endosphincter according to one of the preceding claims, wherein the artificial endosphincter comprises X-ray impermeable markings.

20. An endosphincter according to one of the preceding claims, wherein the retaining piece (2) is provided as a stent, expanding by itself, independent of temperature.

## Revendications

1. Endosphincter (1) artificiel pour l'urètre, avec une pièce de retenue (2) et une soupape (10) pouvant être actionnée manuellement depuis l'extérieur en exerçant une pression, **caractérisé en ce que** la soupape prend la forme d'une soupape cruciforme (10) se fermant automatiquement.

2. Endosphincter selon la revendication 1, **caractérisé en ce que** la soupape cruciforme (10) présente un segment de fixation (11) cylindrique creux et un segment de fermeture (12) orienté vers la pièce de retenue (2).

3. Endosphincter selon la revendication 1 ou 2, **caractérisé en ce que** la soupape cruciforme (10) présente des surfaces d'absorption de pression (13).

4. Endosphincter selon la revendication 3, **caractérisé en ce que** les surfaces d'absorption de pression (13) débouchent dans des lèvres d'étanchéification (14 ; 24-27).

5. Endosphincter selon l'une quelconque des revendications 1 à 4, **caractérisé en ce que** la soupape cruciforme (10) est fabriquée en silicone.

6. Endosphincter selon l'une quelconque des revendications précédentes, **caractérisé en ce que** l'endoprothèse est un grillage (6) tubulaire fabriqué à partir d'au moins un filament.

7. Endosphincter selon la revendication 6, **caractérisé en ce que** la pièce de retenue présente au moins un segment sans grillage tubulaire.

8. Endosphincter selon la revendication 6 ou 7, **caractérisé en ce que** l'au moins un filament présente une section transversale ronde.

9. Endosphincter selon l'une quelconque des revendications 6 à 8, **caractérisé en ce que** l'au moins un filament est fabriqué en matière synthétique ou en métal.

10. Endosphincter selon l'une quelconque des revendications 6 à 9, **caractérisé en ce que** le grillage (6) tubulaire est entouré aussi bien à l'intérieur qu'à l'extérieur par un revêtement qui est lisse sur la surface intérieure et reproduit sur la surface extérieure la structure du grillage.

11. Endosphincter selon la revendication 10, **caractérisé en ce que** le revêtement intérieur prend la forme d'un flexible (5) qui est entouré par le grillage (6) tubulaire.

12. Endosphincter selon la revendication 11, **caractérisé en ce que** le flexible (5) débouche dans un renfoncement (3) de la soupape cruciforme.

13. Endosphincter selon la revendication 12, **caractérisé en ce qu'**une zone de transition (4) entre le renfoncement de soupape (3) et le flexible (5) prend une forme conique.

14. Endosphincter selon la revendication 13, **caractérisé en ce que** la zone de transition (4) est enveloppée par le grillage tubulaire.

15. Endosphincter selon la revendication 14, **caractérisé en ce que** les filaments du grillage tubulaire se rétrécissent en direction du renfoncement de soupape.

16. Endosphincter selon l'une quelconque des revendications 12 à 15, **caractérisé en ce que** le flexible (5) et le renfoncement de soupape (3) sont formés d'un seul tenant, notamment d'un seul tenant en silicone.

17. Endosphincter selon l'une quelconque des revendications précédentes, **caractérisé en ce que** l'endoprothèse prend une forme cylindrique.

18. Endosphincter selon l'une quelconque des revendications précédentes, **caractérisé en ce que** l'endoprothèse présente au moins deux segments cylindriques de rayons différents.

19. Endosphincter selon l'une quelconque des revendications précédentes, **caractérisé en ce que** l'endosphincter artificiel présente des marquages denses aux rayons X.

20. Endosphincter selon l'une quelconque des revendications précédentes, **caractérisé en ce que** la pièce de retenue (2) prend la forme d'une endoprothèse auto-extensible indépendamment de la température.
